# EUROPEAN PATENT APPLICATION

(11) **EP 2 518 159 A1**
(43) Date of publication of application: **31.10.2012**
(21) Application number: 11163980.3
(22) Date of filing: 27.04.2011
(51) Int. Cl.: C12Q 1/68, G01N 33/50

(54) **Method for determining the neurodevelopmental toxicity of a compound in vitro**

(71) Applicant: Universiteit Maastricht, 6211 LK Maastricht (NL); Academisch Ziekenhuis Maastricht, 6229 HX Maastricht (NL); Rijksinstituut Voor Volksgezondheid En Milieu, 3721 MA Bilthoven (NL)
(72) Inventor: Pennings, Jeroen Lambertus Antonius, 3562 TM Utrecht (NL); Piersma, Aldert Henrick, 3720 BA Bilthoven (NL); Theunissen, Petrus Theodorus, 6512 GM Nijmegen (NL)
(74) Representative: Habets, Winand

(57) **Abstract**

This invention is in the field of medical molecular diagnostics. It provides methods and means for the *in vitro* detection of the neurodevelopmental toxicity of a compound by determining the gene expression of a limited number of genes. More in particular, it relates to a method for determining the likelihood that a compound is neurodevelopmental toxic, comprising the steps of: providing embryonic stem cells, allowing the stem cells to form embryoid bodies, allowing the embryoid bodies to differentiate into neural cells, in the presence of said compound, thereby creating a neural differentiation culture, extracting total RNA from the cells in said neural differentiation culture, determining the expression levels of at least one gene selected from the group consisting of genes Car4, Emilin2 and Shisa6, wherein said compound is likely to be neurodevelopmental toxic if the expression level of at least one of these genes is more than 120% or less than 80% of a predetermined reference value.

## Description

### Field of the invention

This invention is in the field of medical molecular diagnostics. It provides methods and means for the *in vitro* detection of the neurodevelopmental toxicity of a compound by determining the gene expression of a limited number of genes.

### Background of the invention

Current chemical hazard assessment for developmental toxicity is built upon globally harmonized OECD (Organization for Economic Co-operation and Development) animal test guidelines, providing a structure for chemical risk assessment and in addition encouraging the development of alternative testing strategies. Under REACH (Regulation, Evaluation and Authorization of Chemicals) guidelines, ∼60% of all animals will be used for reproductive and developmental toxicity studies (van der Jagt et al., 2004).

In order to reduce the number of experimental animals needed for developmental toxicity testing, cell based alternative test methods are being developed, such as embryonic stem cell tests studying multiple differentiation lineages (Genschow et al., 2004; Piersma et al., 2004; Theunissen et al., 2010; zur Nieden et al., 2010), the whole embryo culture (WEC)(Piersma et al., 2004) and the zebrafish embryo test (Hermsen et al., 2011; Nagel, 2002).

An important target for developmental toxicity is the developing nervous system. Classical developmental toxicants, such as methylmercury (MeHg), valproic acid, cyproconazole and ethanol particularly target neural development resulting in neural tube defects, craniofacial malformations, mental retardation and fetal alcohol syndrome (Alsdorf and Wyszynski, 2005; Faustman et al., 2002; Welch-Carre, 2005).

Current *in vitro* test systems for determining neurodevelopmental toxicity depend on assessing cellular endpoints of toxicity such as cell morphology (neural outgrowth, cytotoxicity, migration) (Mundy et al., 2010; Radio and Mundy, 2008) or functional parameters determined by electric conductivity (Shafer et al., 2002).

Regulatory authorities often require that compounds are tested in animals for neurodevelopmental toxicity. Such systems suffer from the disadvantage that they are laborious and costly and require large numbers of animals. In addition, the methods involve behavioral testing which is inherently variable and requires substantial efforts and experimental skills, presupposing high investments in training and equipment. ,

It is an object of the present invention to overcome or ameliorate at least some of the disadvantages of the prior art methods.

### Summary of the invention

It was found that the neurodevelopmental toxicity of a compound could be determined by analyzing the expression level of at least one of three distinct genes in an embryonic stem cell line culture stimulated towards neural differentiation *in vitro.*

The invention therefore relates to a method for determining the likelihood that a compound is neurodevelopmental toxic, comprising the steps of:
a. Providing embryonic stem cells,
b. Allowing the stem cells to form embryoid bodies,
c. Allowing the embryoid bodies to differentiate into neural cells, in the presence of said compound, thereby creating a neural differentiation culture,
d. Extracting total RNA from the cells in said neural differentiation culture,
e. Determining the expression levels of at least one gene selected from the group consisting of genes Car4, Emilin2 and Shisa6,
wherein said compound is likely to be neurodevelopmental toxic if the expression level of at least one of these genes is more than 120% or less than 80% of a predetermined reference value.

### Detailed description of the invention

The present study provides an analysis of differential gene expression in the presence of neurodevelopmental toxicants. This appeared to provide a robust method to monitor neural differentiation in an vitro system wherein embryonic stem cells are allowed to differentiate into neural cells.

The invention therefore relates to a method for determining the likelihood that a compound is neurodevelopmental toxic comprising the steps of:
a. Providing embryonic stem cells,
b. Allowing the stem cells to form embryoid bodies,
c. Allowing the embryoid bodies to differentiate into neural cells, in the presence of said compound, thereby creating a neural differentiation culture,
d. Extracting total RNA from the cells in said neural differentiation culture,
e. Determining the expression levels of at least one gene selected from the group consisting of genes Car4, Emilin2 and Shisa6,
wherein said compound is likely to be neurodevelopmentally toxic if the expression level of at least one of these genes is more than 120% or less than 80% of a predetermined reference value.

The method employs a cell culture assay based on embryonic stem cells. In the examples section we used ES-D3 mouse embryonic stem cells (ESC) obtained from ATCC, Rockville, MD, USA.cultured as detailed in example 2.Any embryonic stem cell culture may be employed in the method according to the invention. A skilled person will know how to adapt the presently exemplified methods to the use of other suitable embryonic stem cell cultures so that it will yield good results in the method according to the invention.

The term "embryonic stem cells" is used herein to mean cells capable of differentiation into diverse specialized cell types that may be isolated from the inner cell mass of blastocysts.

The term "neurodevelopmental toxic" refers to the property of a chemical substance to interfere with the development of neural cells or structures during prenatal development in vertebrates. Disorders considered to be neurodevelopmental in origin or to have neurodevelopmental consequences when they occur in infancy and childhood include autism and autism spectrum disorders such as Asperger syndrome, traumatic brain injury (including congenital injuries such as those that cause cerebral palsy, communication, speech and language disorders, genetic disorders such as fragile-X syndrome, and Down syndrome. Neurodevelopmental disorders are associated with widely varying degrees of mental, emotional, physical and economic burden to individuals, families and society in general.

The term "genes Car4, Emilin2 and Shisa6" is used herein to refer to genes carbonic anhydrase 4, elastin microfibril interfacer 2 and shisa homolog 6, and their homologs in other species.

The term "expression levels" is used herein to mean the level of expression of messenger RNA from a gene. It may be determined by methods such as microarray, real-time PCR or other methods known in the art.

The term "reference value" is used herein to mean the expression level of a gene when measured as described above wherein the neural differentiation culture is not exposed to the compound but otherwise subjected to the same treatment and examined at the same time point. Preferably, the reference value is determined as an average of multiple experiments

When murine embryonic stem cells (ESC) were used in the method according to the invention, it was found to be advantageous to allow the stem cells to form embryoid bodies for about 3 days before contacting them with the compound. The invention therefore relates to a method as described above, wherein step c is carried out 3 days after the start of step b.

In the methods as exemplified herein, it was also found advantageous to allow the neural differentiation culture to grow for one day before isolating total RNA from the differentiating cells. The invention therefore also relates to a method as described above wherein step d is carried out 1 day after step c.

It was found most suitable to induce neural differentiation by incubating the embryoid bodies with a retinoid, preferably retinoic acid. The invention therefore also relates to a method as described above, wherein a retinoid, preferably retinoic acid is used for inducing the embryoid bodies to differentiate into neural cells, thereby establishing a neural differentiation culture.

The method according to the invention provided reliable results with all the test compounds used herein. The gene Emilin2 performed best since it yielded positive results for all compounds tested, genes Car4 and Shisa6 complemented each other and yielded a 100% reliable result when used together. The invention therefore also relates to a method as described above wherein the expression of gene Emilin 2 is determined or wherein the expression of genes Car4 and Shisa6 is determined. Both resulted in a 100% sensitive and accurate result.

The method according to the invention is more reliable than prior art methods, easier to perform, cheaper, and yields results faster.

### Examples

### Example 1: Culture Media

Complete medium (CM) contained Dulbecco's modified eagle's medium (DMEM) (Gibco BRL, Gaithersburg, MD, USA) supplemented with 20% fetal bovine serum (Hyclone, Logan, UT, USA), 1% nonessential amino acids (Gibco BRL, Gaithersburg, MD, USA), 1% penicillin/streptomycin (Gibco BRL, Gaithersburg, MD, USA), 2 mM L-glutamine (Gibco BRL, Gaithersburg, MD, USA) and 0.1 mM â-mercapto ethanol (Sigma-Aldrich, Zwijndrecht, The Netherlands). Low serum medium (LS), had the same composition as CM except that the serum percentage is 10%. Insulin-transferrin-selenite-fibronectin medium (ITS) contained DMEM/Ham's nutrient mixture F12 (DMEM/F12) medium (Gibco, BRL, Gaithersburg, MD, USA) supplemented with 0.2 ug/ml bovine insulin (Sigma-Aldrich, Zwijndrecht, The Netherlands), 1% Penicillin/streptomycin (Gibco BRL, Gaithersburg, MD, USA), 2 mM L-glutamine (Gibco BRL, Gaithersburg, MD, USA), 30 nM sodium selenite (Sigma-Aldrich, Zwijndrecht, The Netherlands), 50 µg/ml apo-transferrin (Sigma-Aldrich, Zwijndrecht, The Netherlands) and 2.5 ug/ml fibronectin (Invitrogen, Carlsbad, CA, USA). N2 medium contained DMEM/F12 medium (Gibco, BRL, Gaithersburg, MD, USA) supplemented with 0.2 ug/ml bovine insulin (Sigma-Aldrich, Zwijndrecht, The Netherlands), 1% penicillin/streptomycin (Gibco BRL, Gaithersburg, MD, USA), 30 nM sodium selenite (Sigma-Aldrich, Zwijndrecht, The Netherlands), 50 µg/ml apo-transferrin (Sigma-Aldrich, Zwijndrecht, The Netherlands).

### Example 2: Embryonic Stem Cell Culture

Murine embryonic stem cells (ESC) (ES-D3, ATCC, Rockville, MD, USA) were routinely subcultured every 2-3 days and grown as a monolayer in CM supplemented with leukemia inhibiting factor (LIF) (Chemicon, Temecula, CA, USA) at a final concentration of 1000 units/ml. The cells were maintained in a humidified atmosphere at 37°C and 5% CO2.

### Example 3: Neural Differentiation Culture

Differentiation of ESC into the neural lineage was carried out as described earlier (Theunissen et al., 2010). In brief, stem cell suspensions (3.75×104 cells/ml) were placed on ice before the initiation of the culture. Drops (20µl) containing 750 cells in CM were placed onto the inner side of the lid of a 90 cm Petri dish filled with phosphate-buffered saline (PBS) (Gibco BRL, Gaithersburg, MD) and incubated at 37 °C, 90% relative humidity and 5% CO2. After 3 days of hanging drop culture embryoid bodies (EB) had formed and were subsequently transferred to 60mm bacterial Petri dishes (Greiner Bio-one, Frickenhausen, Germany) containing CM supplemented with 0.5 µM retinoic acid (RA). On day 5, EB were plated on laminin (Roche, Basel, Switzerland) coated dishes (Corning Incorporated, Corning, NY, USA) in LS medium supplemented with 2.5 ug/ml fibronectin (Invitrogen, Carlsbad, CA, USA). One day later, on day 6, the LS medium was replaced by ITS medium. On day 7, EB were washed with phosphate buffered saline (PBS) (Gibco BRL, Gaithersburg, MD, USA) and incubated in cell dissociation buffer (Gibco BRL, Gaithersburg, MD, USA) for 3 minutes. Then EB were carefully detached from each Petri dish without dissociating the EBs and the entire content was replated on poly-L-ornithine (Sigma-Aldrich, Zwijndrecht, The Netherlands) and laminin coated dish in N2 medium supplemented with 10 ng/ml basic fibroblast growth factor (bFGF) (Srtathmann-Biotec AG, Englewood, CO, USA). The N2 medium supplemented with bFGF was replaced every other day for 7 days.

### Example 4: Treatment and morphological scoring for effects on neural outgrowth

Methylmercury chloride (CAS number 115-09-3, Sigma-Aldrich, Zwijndrecht, The Netherlands) was diluted in DMSO and added to the medium to final concentrations in culture of 25 nM MeHg.

Cyproconazole (CAS number 94361-06-5, Sigma-Aldrich, Zwijndrecht, The Netherlands) was diluted in DMSO and added to the medium to final concentrations in culture of 100 µM cyproconazole.

Hexaconazole (CAS number 79983-71-4, Sigma-Aldrich, Zwijndrecht, The Netherlands) was diluted in DMSO and added to the medium to final concentrations in culture of 25 µM hexaconazole.

Valproic acid sodium salt (CAS number 1069-66-5, Sigma-Aldrich, Zwijndrecht, The Netherlands) was diluted in DMSO and added to the medium to final concentrations in culture of 1000 µM valproic acid.

Control EB were treated with 0.01 % DMSO as described earlier from day 3 of the protocol until day 11 (Theunissen et al., 2010). Effects were determined by assessing of the morphological extent of neural outgrowth, at day 11, observed using an IX51 inverted microscope (Olympus, Zoeterwoude, The Netherlands) with CellD software (Olympus, Zoeterwoude, The Netherlands). Morphological neural outgrowth was scored as <75% or =75% of the neural corona surrounding each EB, irrespective of the distance of outgrowth from the EB. Parallel cultures were harvested for gene expression analysis at earlier time points.

### Example 5: RNA isolation and whole-genome expression profiling

Control differentiation cultures were harvested on culture days 3, 4 and 5 (8 replicates per group). Differentiation cultures were exposed to the 3 neurodevelopmental toxicants and controls from day 3 onwards and harvested on day 4 (24h exposure) (8 replicates per group). Cells (-40-50 EB/sample) were directly collected in RNA Protect (Qiagen Benelux, Venlo, The Netherlands) to stabilize RNA, and total RNA was purified using the Qiagen RNEasy Plus Mini Kit (Qiagen Benelux, Venlo, The Netherlands) following the manufacturer's instructions. RNA quantity was determined using the NanoDrop Spectophotometer (Isogen Lifescience, de Meern, The Netherlands). RNA integrity was assessed on the 2100 Bioanalyzer (Agilent, Santa Clara, CA, USA) using the RNA 6000 Nano Chip Kit (Agilent, Santa Clara, CA, USA), and good quality RNA was used for gene expression analysis (RNA integrity number (RIN) > 8.0). Labeled aRNA target was generated by using the Affymetrix genechip (Affymetrix, Santa Clara, CA, US) (3'IVT express kit 4x24 reactions (P/N 901225) according to the instructions in the user manual. Briefly, 250 ng of each total RNA sample and also controls were used to prepare first-strand cDNA. After making the complementary second strand, the double stranded cDNA is amplified by *in vitro* transcription (IVT). During this IVT reaction a biotinylated nucleotide analog is incorporated in the aRNA. This IVT reaction was followed by aRNA purification with magnetic beads and fragmentation (15 µg) of each aRNA sample. The whole process from first-strand cDNA synthesis till fragmentation of the aRNA was performed by the Xiril Neon 150 robotic system (GC-Biotech, Alphen aan den Rijn, The Netherlands).

After fragmentation, a 250 µl hybridization cocktail with 12,5 µg of fragmented aRNA was prepared. 200 µl of this cocktail (10 µg aRNA target) was applied to the Mouse Genome 430 2.0 arrays (P/N 900497, Affymetrix, Santa Clara, CA, US) and hybridized for 16 hours at 45°C in a Genechip Hybridization Oven 640. After hybridization the arrays were washed and stained with a Genechip Fluidics Station 450 using the Affymetrix genechip hybridization wash and stain kit (P/N 900720). Washed and stained arrays were scanned using the genechip scanner 3000 from Affymetrix. Hybridization was performed at Affymetrix (Santa Clara, CA, US).

### Example 6: Data Analysis and Statistics

Gene expression levels were determined as normalized probe set intensities, as is custom for Affymetrix arrays. To this end, Affymetrix CEL files were normalized using the Robust Multichip Average (RMA) algorithm (Irizarry et al., 2003) using RMAexpress (Bolstad et al., 2003). For probe to gene mapping, a custom Chip Description File (CDF) was used according to the assembly by de Leeuw et al. (de Leeuw et al., 2008) (http://mad-db.science.uva.nl/∼wdeleeuw/HybridAnnot/version6.html). Of the hybrid probe-set definitions included in the custom annotation, 16,331 probe sets defined by the Brainarray custom CDF version 11 http://brainarray.mbni.med.umich.edu/Brainarray/Database/CustomCDF) (Dai et al., 2005) and 4648 additional probe sets defined by Affymetrix chip annotation 26 were used in further analyses, giving a total of 20,979 probe sets. Probe sets for Affymetrix internal controls or probe sets that did not correspond to an Entrez Gene ID were not used in further analyses. Expression changes were calculated comparing the normalized signal of the compound-exposed samples relative to the average normalized signal of the DMSO-exposed samples (control). Table values (FR) of 1 equals unchanged compared to the control. Values less than 1 indicate downregulation compared to the control, values larger than 1 indicate upregulation.

**Table 1. Gene expression changes for the three biomarker genes.**

| | | | |
|---|---|---|---|
| Mouse GeneID | 12351 | 246707 | 380702 |
| Mouse gene symbol | Car4 | Emilin2 | Shisa6 |
| Ratio MeHg | 0.686 | 1.351 | 1.380 |
| Ratio Cyp | 0.777 | 1.270 | 1.228 |
| Ratio VPA | 1.373 | 2.063 | 0.752 |
| Ratio Hex | 0.936 | 1.185 | 1.096 |
| human homolog GeneID | 762 | 84034 | 388336 |
| human homologgene symbol | CA4 | EMILIN2 | SHISA6 |
| rat homolog GeneID | 29242 | 316736 | 497926 |
| rat homolog gene symbol | Car4 | Emilm2 | Shisa6 |

### References

Van der Jagt, K., Tørsløv, J. and de Bruijn, J. (2004). Alternative approaches can reduce the use of test animals under REACH., Ed.^ Eds.). European Commission Report.
Genschow, E., Spielmann, H., Scholz, G., Pohl, I., Seiler, A., Clemann, N., Bremer, S. and Becker, K. (2004). Validation of the embryonic stem cell test in the international ECVAM validation study on three in vitro embryotoxicity tests. Altern Lab Anim 32, 209-44.
Piersma, A. H., Genschow, E., Verhoef, A., Spanjersberg, M. Q., Brown, N. A., Brady, M., Burns, A., Clemann, N., Seiler, A. and Spielmann, H. (2004). Validation of the postimplantation rat whole-embryo culture test in the international ECVAM validation study on three in vitro embryotoxicity tests. Altern Lab Anim 32, 275-307.
Theunissen, P. T., Schulpen, S. H., van Dartel, D. A., Hermsen, S. A., van Schooten, F. J. and Piersma, A. H. (2010). An abbreviated protocol for multilineage neural differentiation of murine embryonic stem cells and its perturbation by methyl mercury. Reprod Toxicol 29, 383-92,
zur Nieden, N. I., Davis, L. A. and Rancourt, D. E. (2010). Comparing three novel endpoints for developmental osteotoxicity in the embryonic stem cell test. Toxicol Appl Pharmacol 247, 91-7,
Hermsen, S. A., van den Brandhof, E. J., van der Ven, L. T. and Piersma, A. H. (2011). Relative embryotoxicity of two classes of chemicals in a modified zebrafish embryotoxicity test and comparison with their in vivo potencies. Toxicol In vitro*,* Nagel, R. (2002). DarT: The embryo test with the Zebrafish Danio rerio--a general model in ecotoxicology and toxicology. ALTEX 19 Suppl 1, 38-48.
Alsdorf, R. and Wyszynski, D. F. (2005). Teratogenicity of sodium valproate. Expert Opin Drug Saf 4, 345-53,].
Faustman, E. M., Ponce, R. A., Ou, Y. C., Mendoza, M. A., Lewandowski, T. and Kavanagh, T. (2002). Investigations of methylmercury-induced alterations in neurogenesis. Environ Health Perspect 110 Suppl 5, 859-64,
Welch-Carre, E. (2005). The neurodevelopmental consequences of prenatal alcohol exposure. Adv Neonatal Care 5, 217-29, S1536-0903(05)00148-7 [pii] 10.1016/j.adnc.2005.04.007.
Costa, L. G., Fattori, V., Giordano, G. and Vitalone, A. (2007). An in vitro approach to assess the toxicity of certain food contaminants: methylmercury and polychlorinated biphenyls. Toxicology 237, 65-76,
Shafer, T. J., Meacham, C. A. and Barone, S., Jr. (2002). Effects of prolonged exposure to nanomolar concentrations of methylmercury on voltage-sensitive sodium and calcium currents in PC12 cells. Brain Res Dev Brain Res 136, 151-64,
Stummann, T. C., Hareng, L. and Bremer, S. (2007). Embryotoxicity hazard assessment of methylmercury and chromium using embryonic stem cells. Toxicology 242, 130-43,
Walum, E. and Flint, O. P. (1990). Midbrain micromass cultures: a model for studies of teratogenic and sub-teratogenic effects on CNS development. Acta Physiol Scand Suppl 592, 61-72.
Bibel, M., Richter, J., Schrenk, K., Tucker, K. L., Staiger, V., Korte, M., Goetz, M. and Barde, Y. A. (2004). Differentiation of mouse embryonic stem cells into a defined neuronal lineage. Nat Neurosci 7, 1003-9,
Okabe, S., Forsberg-Nilsson, K., Spiro, A. C., Segal, M. and McKay, R. D. (1996). Development of neuronal precursor cells and functional postmitotic neurons from embryonic stem cells in vitro. Mech Dev 59, 89-102,
Mundy, W. R., Radio, N. M. and Freudenrich, T. M. (2010). Neuronal models for evaluation of proliferation in vitro using high content screening. Toxicology 270, 121-30,
Radio, N. M. and Mundy, W. R. (2008). Developmental neurotoxicity testing in vitro: models for assessing chemical effects on neurite outgrowth. Neurotoxicology 29, 361-76,
Robinson, J. F., Griffith, W. C., Yu, X., Hong, S., Kim, E. and Faustman, E. M. (2010a). Methylmercury induced toxicogenomic response in C57 and SWV mouse embryos undergoing neural tube closure. Reprod Toxicol 30, 284-91,
Robinson, J. F., Guerrette, Z., Yu, X., Hong, S. and Faustman, E. M. (2010b). A systems-based approach to investigate dose- and time-dependent methylmercury-induced gene expression response in C57BL/6 mouse embryos undergoing neurulation. Birth Defects Res B Dev Reprod Toxicol 89, 188-200,.
Robinson, J. F., van Beelen, V. A., Verhoef, A., Renkens, M. F., Luijten, M., van Herwijnen, M. H., Westerman, A., Pennings, J. L. and Piersma, A. H. (2010c). Embryotoxicant-specific transcriptomic responses in rat postimplantation whole-embryo culture. Toxicol Sci 118, 675-85,
Krewski, D. (2007). Toxicity Testing in the Twenty-first Century (C. o. T. a. A. o. E. A. NRC, Ed.^ Eds.). National Academy of Sciences, Washington, DC.
Nemeth, K. A., Singh, A. V. and Knudsen, T. B. (2005). Searching for biomarkers of developmental toxicity with microarrays: normal eye morphogenesis in rodent embryos. Toxicol Appl Pharmacol 206, 219-28,
van Dartel, D. A., Pennings, J. L., de la Fonteyne, L. J., Brauers, K. J., Claessen, S., van Delft, J. H., Kleinjans, J. C. and Piersma, A. H. (2011). Evaluation of developmental toxicant identification using gene expression profiling in embryonic stem cell differentiation cultures. Toxicol Sci 119, 126-34,
van Dartel, D. A., Pennings, J. L., de la Fonteyne, L. J., van Herwijnen, M. H., van Delft, J. H., van Schooten, F. J. and Piersma, A. H. (2010a). Monitoring developmental toxicity in the embryonic stem cell test using differential gene expression of differentiation-related genes. Toxicol Sci 116, 130-9,
van Dartel, D. A., Pennings, J. L., van Schooten, F. J. and Piersma, A. H. (2010b). Transcriptomics-based identification of developmental toxicants through their interference with cardiomyocyte differentiation of embryonic stem cells. Toxicol Appl Pharmacol 243, 420-8,
Cox, C., Clarkson, T. W., Marsh, D. O., Amin-Zaki, L., Tikriti, S. and Myers, G. G. (1989). Dose-response analysis of infants prenatally exposed to methyl mercury: an application of a single compartment model to single-strand hair analysis. Environ Res 49, 318-32.
Davidson, P. W., Myers, G. J. and Weiss, B. (2004). Mercury exposure and child development outcomes. Pediatrics 113, 1023-9.
Burbacher, T. M., Rodier, P. M. and Weiss, B. (1990). Methylmercury developmental neurotoxicity: a comparison of effects in humans and animals. Neurotoxicol Teratol 12, 191-202,
Myers, G. J. and Davidson, P. W. (1998). Prenatal methylmercury exposure and children: neurologic, developmental, and behavioral research. Environ Health Perspect 106 Suppl 3, 841-7.
Irizarry, R. A., Hobbs, B., Collin, F., Beazer-Barclay, Y. D., Antonellis, K. J., Scherf, U. and Speed, T. P. (2003). Exploration, normalization, and summaries of high density oligonucleotide array probe level data. Biostatistics 4, 249-64,
Bolstad, B. M., Irizarry, R. A., Astrand, M. and Speed, T. P. (2003). A comparison of normalization methods for high density oligonucleotide array data based on variance and bias. Bioinformatics 19, 185-93.
de Leeuw, W. C., Rauwerda, H., Jonker, M. J. and Breit, T. M. (2008). Salvaging Affymetrix probes after probe-level re-annotation. BMC Res Notes 1, 66,
Dai, M., Wang, P., Boyd, A. D., Kostov, G., Athey, B., Jones, E. G., Bunney, W. E., Myers, R. M., Speed, T. P., Akil, H., Watson, S. J. and Meng, F. (2005). Evolving gene/transcript definitions significantly alter the interpretation of GeneChip data. Nucleic Acids Res 33,
Huang da, W., Sherman, B. T., Tan, Q., Collins, J. R., Alvord, W. G., Roayaei, J., Stephens, R., Baseler, M. W., Lane, H. C. and Lempicki, R. A. (2007). The DAVID Gene Functional Classification Tool: a novel biological module-centric algorithm to functionally analyze large gene lists. Genome Biol 8, R183,
Boorsma, A., Foat, B. C., Vis, D., Klis, F. and Bussemaker, H. J. (2005). T-profiler: scoring the activity of predefined groups of genes using gene expression data. Nucleic Acids Res 33,
Kuegler, P. B., Zimmer, B., Waldmann, T., Baudis, B., Ilmjarv, S., Hescheler, J., Gaughwin, P., Brundin, P., Mundy, W., Bal-Price, A. K., Schrattenholz, A., Krause, K. H., van Thriel, C., Rao, M. S., Kadereit, S. and Leist, M. (2010). Markers of murine embryonic and neural stem cells, neurons and astrocytes: reference points for developmental neurotoxicity testing. ALTEX 27, 17-42.
Su, A. I., Wiltshire, T., Batalov, S., Lapp, H., Ching, K. A., Block, D., Zhang, J., Soden, R., Hayakawa, M., Kreiman, G., Cooke, M. P., Walker, J. R. and Hogenesch, J. B. (2004). A gene atlas of the mouse and human protein-encoding transcriptomes. Proc Natl Acad Sci U S A 101,
Pearson, K. (1901). On lines and planes of closest fit to systems of points in space. Philos. Mag. 2, 559-72.
Niwa, H., Miyazaki, J. and Smith, A. G. (2000). Quantitative expression of Oct-3/4 defines differentiation, dedifferentiation or self-renewal of ES cells. Nat Genet 24, 372-6,
Ogawa, K., Saito, A., Matsui, H., Suzuki, H., Ohtsuka, S., Shimosato, D., Morishita, Y., Watabe, T., Niwa, H. and Miyazono, K. (2007). Activin-Nodal signaling is involved in propagation of mouse embryonic stem cells. J Cell Sci 120, 55-65,
Liu, L., Geisert, E. E., Frankfurter, A., Spano, A. J., Jiang, C. X., Yue, J., Dragatsis, I. and Goldowitz, D. (2007). A transgenic mouse class-III beta tubulin reporter using yellow fluorescent protein. Genesis 45, 560-9,.
Moskowitz, P. F. and Oblinger, M. M. (1995). Transcriptional and post-transcriptional mechanisms regulating neurofilament and tubulin gene expression during normal development of the rat brain. Brain Res Mol Brain Res 30, 211-22,
Abu-Abed, S., Dolle, P., Metzger, D., Beckett, B., Chambon, P. and Petkovich, M. (2001). The retinoic acid-metabolizing enzyme, CYP26A1, is essential for normal hindbrain patterning, vertebral identity, and development of posterior structures. Genes Dev 15, 226-40.
Abranches, E., Silva, M., Pradier, L., Schulz, H., Hummel, O., Henrique, D. and Bekman, E. (2009). Neural differentiation of embryonic stem cells in vitro: a road map to neurogenesis in the embryo. PLoS One 4, e6286, 10.
Kim, M., Habiba, A., Doherty, J. M., Mills, J. C., Mercer, R. W. and Huettner, J. E. (2009). Regulation of mouse embryonic stem cell neural differentiation by retinoic acid. Dev Biol 328, 456-71,]
Zimmer, B., Kuegler, P. B., Baudis, B., Genewsky, A., Tanavde, V., Koh, W., Tan, B., Waldmann, T., Kadereit, S. and Leist, M. (2010). Coordinated waves of gene expression during neuronal differentiation of embryonic stem cells as basis for novel approaches to developmental neurotoxicity testing. Cell Death Differ, cdd2010109 [pii]
Mitiku, N. and Baker, J. C. (2007). Genomic analysis of gastrulation and organogenesis in the mouse. Dev Cell 13, 897-907,
Ceccatelli, S., Dare, E. and Moors, M. (2010). Methylmercury-induced neurotoxicity and apoptosis. Chem Biol Interact 188, 301-8,
Spyker, J. M. and Smithberg, M. (1972). Effects of methylmercury on prenatal development in mice. Teratology 5, 181-90,.
Pellizzer, C., Adler, S., Corvi, R., Hartung, T. and Bremer, S. (2004). Monitoring of teratogenic effects in vitro by analysing a selected gene expression pattern. Toxicol In vitro 18, 325-35,
van Dartel, D. A., Pennings, J. L., Hendriksen, P. J., van Schooten, F. J. and Piersma, A. H. (2009). Early gene expression changes during embryonic stem cell differentiation into cardiomyocytes and their modulation by monobutyl phthalate. Reprod Toxicol 27, 93-102,
Genschow, E., Spielmann, H., Scholz, G., Seiler, A., Brown, N., Piersma, A., Brady, M., Clemann, N., Huuskonen, H., Paillard, F., Bremer, S. and Becker, K. (2002). The ECVAM international validation study on in vitro embryotoxicity tests: results of the definitive phase and evaluation of prediction models. European Centre for the Validation of Alternative Methods. Altern Lab Anim 30, 151-76.

## Claims

1. Method for determining the likelihood that a compound is neurodevelopmental toxic, comprising the steps of:
a. Providing embryonic stem cells,
b. Allowing the stem cells to form embryoid bodies,
c. Allowing the embryoid bodies to differentiate into neural cells, in the presence of said compound, thereby creating a neural differentiation culture,
d. Extracting total RNA from the cells in said neural differentiation culture,
e. Determining the expression levels of at least one gene selected from the group consisting of genes Car4, Emilin2 and Shisa6,
wherein said compound is likely to be neurodevelopmental toxic if the expression level of at least one of these genes is more than 120% or less than 80% of a predetermined reference value.

2. Method according to claim 1 wherein step c is carried out 3 days after the start of step b.

3. Method according to claims 1 or 2 wherein step d is carried out 1 day after step c.

4. Method according to claims 1-3 wherein a retinoid, preferably retinoic acid is used for inducing the embryoid bodies to differentiate into a neural lineage.

5. Method according to claims 1-4 wherein the expression level of said genes is determined by quantitative amplification of nucleic acid.

6. Method according to claim 5 wherein said amplification of nucleic acid includes a polymerase chain reaction.

7. Method according to claims 1-6 wherein the expression of gene Emilin2 is determined.

8. Method according to claims 1-7 wherein the expression level of genes Car4 and Shisa6 is determined.
